Europäisches Patentamt

European Patent Office

Office européen des brevets

(11) Publication number: **0 243 553**
**A1**

## (12) EUROPEAN PATENT APPLICATION

(21) Application number: 86307774.9

(22) Date of filing: 08.10.86

(51) Int. Cl.⁴: **C12N 15/00 , A01H 1/00**

(30) Priority: 25.04.86 US 855674

(43) Date of publication of application:
04.11.87 Bulletin 87/45

(84) Designated Contracting States:
**AT BE CH DE ES FR GB GR IT LI LU NL SE**

(71) Applicant: **SUNGENE TECHNOLOGIES CORPORATION**
**3330 Hillview Avenue**
**Palo Alto California 94304(US)**

(72) Inventor: **Park, F. Dale**
**4619 Norwalk Street, Union City**
**California 94586(US)**
Inventor: **Hicks, Glenn R.**
**283 Crown Circle, San Francisco**
**California 94080(US)**
Inventor: **Cheng, David S.K.**
**825 Vespucci Lane, Foster City**
**California 94404(US)**

(74) Representative: **Bannerman, David Gardner et al**
**Withers & Rogers 4 Dyer's Buildings Holborn**
**London, EC1N 2JT(GB)**

(54) **Plant transformation vector.**

(57) The present invention relates to a plant transformation vector which is capable of transforming monocots and dicots. The plant transformation vector comprises the Mu1 element and foreign DNA. The vector may further contain a marker. The foreign DNA and/or marker is positioned entirely within the Mu1 element or adjacent the Mu1 element. The present invention also relates to a recombinant vector useful for transforming plants which contains a DNA insert consisting essentially of the Mu1 element.

EP 0 243 553 A1

## PLANT TRANSFORMATION VECTOR

### BACKGROUND OF THE INVENTION

#### Field of the Invention

The present invention relates to a plant transformation vector which is capable of introducing foreign DNA into both monocots and dicots. More particularly, the invention relates to a vector which comprises a DNA sequence corresponding to a substantial portion of the DNA sequence of the Mu1 element of maize. The invention further relates to a process for transforming plants utilizing this vector.

#### Description of the Prior Art

Several approaches for the introduction of foreign DNA into plants are currently being developed or perfected. Transformation of plant cells mediated by infection by Agrobacterium tumefaciens and subsequent transfer of a region of plasmid DNA, referred to as T-DNA, have been well documented. See, for example, Bevan, M. W., et al., Ann.Rev.Genet. 16 , 357 (1982). A. tumefaciens is the etiologic agent of crown gall, which is a disease of a wide range of dicotyledons and gymnosperms (DeCleene, M., et al., Botan. Rev. 42, 389 (1976)) that results in the formation of tumors or galls in plant tissue at the site of infection. This bacterium, which normally infects at wound sites, carries a large extra chromosomal element called the Ti plasmid (for tumor inducing) which in turn contains two regions required for tumorogenicity. This is the T-DNA (for transfer DNA) which is actually transferred into the plant cells and is incorporated into the plant genome and the vir region (for virulence) which has been implicated in the transfer mechanism.

Ti plasmids cane be manipulated as gene vectors and have been used for the stable introduction and expression of foreign genes in plant cells and regenerated plants. See, for example, Fraley, R. T., et al., Proc.Nat.Acad.Sci.USA 84, 4803 (1983); Herrera-Estrella, L., et al.,EMBO J 2, 987 (1983); Helmer, G., et al., Bio/Technology 2, 520 (1984); and Murai, N., et al., Science 222, 476 (1983). This work has principally been confined to tobacco and petunia protoplasts and explants. Tomato, sunflower, potato and, to a lesser extent, soybean (Facciotti, D., et al., Bio/Technology 3 , 241 (1985)) have also been used. However, transformed soybean plants have not been obtained prior to the present invention. Tobacco has been the host of choice due to its ease of manipulation. Several laboratories have been engaged in modifying the Ti plasmid, with emphasis on the T-DNA, in such a fashion as to permit more ready selection of transformed tissue. This modification has included fusing the promoters of the opine synthase genes for nopaline or octopine with bacterial antibiotic resistance genes. Other promoters, such as those for the cauliflower mosaic virus (CaMV) 35S RNA (Odell, J. T., et al., Nature 313, 810 (1985)) and ribulose-1,5-biphosphate-carboxylase from soybean and pea (Fuccliotti, D., et al., supra, Broglie, R., et al., Science 244, 838 (1984)), have also been used successfully. Other structural genes, such as that for $\beta$-galactosidase from Escherichia coli - (Helmer, G., et al., supra) and the bean storage protein, phaseolin (Murai, N., et al., supra), have been successfully fused to active plant promoters and have been expressed. The preferred package for the foreign gene has been termed a cassette or chimeric gene. In this case, the package comprises a plant expressible promoter, a foreign structural gene, and plant-recognized 3' sequences. Intensive study of the T-DNA region has led to several significant discoveries related to its organization. The T-DNA is bordered by two imperfect direct repeats of 25 nucleotides which have been implicated as sites of transfer for this region. Wang, K., et al., Cell 38, 455 (1984). For example, the right border 25 base pair repeat of nopaline T-DNA appears to be capable of mediating the transfer of large stretches of DNA positioned to its upstream side without the left border repeat, which normally serves as its transfer terminus. Caplan, et al., J. Bact. 61, 655 (1985). The transfer mechanism does not seem to differentiate between sequences located adjacent the right border. This permits the disruption or deletion of the tumorogenic loci, tms and tmr, without deleterious effect. Zambryski, P., et al., EMBO J 2, 2143 (1983). Also, the vir region functions can act in trans to mobilize the T-DNA region. Hille, J., et al., J.Bact., 158, 754 (1984). These discoveries have led to the construction of a range of transformation vectors which are non-oncogenic, thereby making possible the infection and transformation of tissue retaining the characteristics of normally differentiating cells which may be regenerable into whole plants. See De Block, M., et al., EMBO J 3, 1681 (1984) and Barton, K. A., et al., Cell 32, 1033 (1983). Binary vector systems have been developed in which the T-DNA region is present on a smaller, more easily manipulated plasmid and the vir region retained on a modified, disarmed Ti plasmid. See, for example, Klee, H. J., et al., Bio/Technology 3, 637 (1985) and Fraley, R. T., et al., Biotechnology 3,. 629 (1985). Any gene sequence may be placed between the borders of the T-DNA including signals for the expression of the gene in a specific tissue or developmental stage. The possibilities, then, of transformation of plants using this system, seem to be limited only by the host range of Agrobacterium and the ability to regenerate the transformed tissue.

Although this system has been attempted in order to transform monocots, it has generally met with very limited success. Thus, Narcissus and Chlorophytum produce swellings that contain opines following inoculation with A . tumefaciens --expression of opine synthesis genes usually depends on their transfer into plant cells. And the induction of tumorous growth in Aparagus plants by A. tumefaciens with the production of nopaline demonstrates that some monocotyledonous plants are susceptible to infection by Agrobacterium and can express tumor DNA, although conclusive proof of the integration of foreign DNA is lacking. Yet the genetic transformation of monocots remains a most important goal in plant genetic engineering, for many of the world's major food crops are monocots --rice, barley, maize, wheat and sorghum, for example. The many claims that "transformed" monocot plants have been obtained after the microinjection or uptake of DNA into immature or mature seeds have been based on the observation of altered plant phenotypes, but have not been supported by the more definite proofs that can be gained using molecular biology. Much effort is now being directed towards giving such proof.

One approach which has been developed to transform both monocots and dicots is the direct gene transfer method. In this method, a gene is directly introduced into a protoplast. The gene is then incorporated into the nuclear genome. This approach avoids the need for introduction of cloned DNA into the T-DNA of theA. tumefaciens Ti plasmid or its equivalent. The early work was conducted in tobacco, as reported in Paszkowski, J., et al., EMBO J 3, 2717 (1984), and Potrykus, I., et al., Mol.Gen.Genet. 199, 169 (1985). A selectable foreign DNA was introduced into protoplasts by direct gene transfer, where it became integrated into the nuclear genome. This DNA is stably incorporated and is transmitted to progeny plants in a mendelian fashion. The direct gene transfer approach has been used routinely to transform animal cells; it is surprising that this method has not been more widely attempted for plant cells. One reason is that plant protoplasts, required for the method, are more fragile and more difficult to culture than many animal cells. In particular, as a result of the trauma of isolation, plant protoplast DNA synthesis and mitoses are usually delayed for some days following isolation. Integration of foreign DNA may occur during genome DNA synthesis, so the introduced DNA may be degraded by nucleases before it can be integrated.

Monocot protoplasts have also been transformed by this procedure. As described by Lorz, H., et al., Mol.Gen.Genet. 199, 178 (1985), DNA from a selectable plasmid (pBL1103-4, containing a selectable chimeric gene with the nopaline synthase promoter, the coding region of Tn5 aminoglycoside phosphotransferase type II gene (NPT II), the polyadenylation signal of the octopine synthase gene and a maize transposable element, Ac) was introduced into protoplasts isolated from suspension cultures of Triticum monococcum. Transformed cells were selected and characterized by detection of aminoglycoside phosphotransferase II activity. The transformation frequency was about 1 in 5 x $10^6$ protoplasts. The Ac element was included in the introduced DNA to study its possible activation after integration in a cereal other than maize.

Similar transfer was performed by Potrykus, I., et al., Mol.Gen. Genet. 199, 183 (1985) in Lolium multiflorum (Italian ryegrass). In this work, the selectable plasmid (pABDl), consisting of a chimeric construction in which the aminoglycoside phosphotransferase gene of transposon Tn5 is under the control of promoter, terminator and initiation signals from gene VI of cauliflower mosaic virus, was introduced into protoplast recipients obtained from suspension cultures of Lolium multiflorum. Cell colonies resistant to antibiotic G418 were selected (untransformed ryegrass suspension protoplasts were resistant to relatively high concentrations of kanamycin). The active product of the phosphotransferase gene was found in these selected cells. In addition, genomic DNA from these lines was extracted and probed with the nick-translated gene by Southern blot analysis, and the presence of at least five copies per genome of the introduced DNA was detected, apparently without any further rearrangements. Thus the stable integration of a foreign gene into a gramineous species has been achieved. In this case, the transformation frequency is about 1 in 4 x $10^6$.

Similar methods were used in both cases to introduce the DNA into the protoplasts. Following linearization of the plasmid DNA, its introduction into the protoplasts was accomplished by incubation of protoplasts and DNA in the presence of polyethylene glycol with or without excess carrier DNA, using procedures developed by Krens, F.A., et al., Nature 296 , 72 (1982).

In a report to the First International Congress of Plant Molecular Biology (October 27 - November 2, 1985), Fromm, M., et al. disclosed that they had transformed Black Mexican sweet corn protoplasts via electroporation with a vector that contained a construct of CaMV 35S promoter, NPT II gene and nos polyA site. Calli were isolated which were Kan$^R$ and shown to be maize cells by Adh activity. Southern blot showed that the NPT II gene was present in the calli.

A third approach which has been suggested is to utilize plant transposable elements for plant transformation. See, for example, Thomas, T. L., et al., Bio.Essays 3, 149 (1985), and Doring, H-P., Bio.Essays 3, 164 (1985). Several plant transposable elements are known, especially in maize. These include the Ac-Ds system, Mu1, Tz86 and Spm-En. The DNA sequence has been determined for Ds and Mu1 by Doring, H-P., et al., Nature 307, 127 (1984) and Barker, R. F., et al., Nucl.Acids Res. 12, 5955 (1984), respectively. Thomas, T. L., et al., supra, note that Mu1 appears to show considerable promise as a means for transposon mutagenesis and marker rescue. However, Mu1 contains densely packed open-reading frames in its DNA sequence, which suggests that it may be difficult to develop as a vector. The present invention is the first instance of the preparation of plant transformation vectors which contain Mu1 and the use of these vectors to transform plants.

## SUMMARY OF THE INVENTION

The present invention is directed to a plant transformation vector which comprises the Mu1 element and foreign DNA. The foreign DNA is inserted entirely within the Mu1 element or entirely on the 5′ or 3′ side of the Mu1 element in the vector.

More specifically, the plant transformation vector is a plasmid into which the Mu1 element has been inserted. It is preferred to modify the Mu1 element by inserting into it a DNA fragment which contains sequences recognized by several different restriction endonucleases. This DNA fragment will be referred to hereinafter as a multiple cloning site. Foreign DNA can be cloned entirely into the multiple cloning site or into any alternative restriction site within the Mu1 element. Alternatively, the foreign DNA can be cloned entirely on the 5′ or 3′ side of the Mu1 element in the vector.

The present invention is further directed to a vector which is useful in the transformation of plants, which comprises a cloning vector having covalently bound thereto a DNA insert consisting essentially of the DNA sequence of the Mu1 element of maize.

The present invention is also directed to the specific plasmids pM10, pM11, pM12, pM14 or modifications thereof. "Modifications" refers to alterations in these plasmids which are within the skill in the art.

The present invention is further directed to a process for transforming plants. In this process, the plant transformation vector is introduced into plant cells. The foreign DNA is integrated into the plant cell genome by the transposable element function of Mu1. The plant cells are grown and selected for transformants. Plants are then regenerated from the transformed cells.

## BRIEF DESCRIPTION OF THE FIGURES

Figure 1 shows the construction of pM10 and pM11, in which the Mu1 element is shown as a closed box.

Figure 2 shows the construction of pM12, in which the Mu1 element is shown as a closed box.

Figure 3 shows the construction of pM14, in which the Mu1 element is shown as a closed box.

Figure 4 is a drawing of the gel pattern for kanamycin assay of transformed tobacco callus.

Figure 5 is a drawing of the gel pattern for a kanamycin assay of transformed tobacco tissue.

## DETAILED DESCRIPTION OF THE INVENTION

The present invention is directed to a plant transformation vector which is capable of transforming both monocots and dicots. Foreign DNA is inserted into the vector in order to transfer the foreign DNA to plants and to transform the plants. The term "foreign DNA" is used herein to refer to DNA which is naturally exogenous to the plants or naturally not found in the plants which are to be transformed. Such foreign DNA includes prokaryotic DNA as well as eukaryotic DNA, and may be naturally occurring DNA, chemically synthesized DNA, cDNA, mutated DNA or any combination of the same.

The present invention is further directed to a recombinant vector which is useful in the transformation of plants. The recombinant vector may be utilized to co-transform plants with a transformation vector containing the desired foreign DNA. The recombinant vector contains the Mu1 element from maize. The vector may be any vector, such as a plasmid or virus, which is capable of replication in a host selected for the initial construction of the vectors and for replication of the vectors. It is presently preferred to utilize Escherichia coli, although any suitable prokaryotic or eukaryotic host may be used.

A starting vector is selected for use in the chosen host. For E . coli, these vectors may include pBR322, pUC8, pGEM1, among others. The recombinant vector and plant transformation vector are constructed using techniques well known in the art. Suitable techniques have been described in Maniatis, T., et al., Molecular Cloning, Cold Spring Harbor Laboratory, New York (1982). Medium compositions have been described in Miller, J. H., Experiments in Molecular Genetics, Cold Spring Harbor Laboratory, New York (1972). The Mu1 element is inserted into the starting vector via conventional techniques to produce the recombinant vector. For example, the Mu1 element is removed from plasmid pMJ9 (obtained from M. J. Freeling, University of California, Berkeley) by digestion with HindIII, Sau3a, and PvuII. The Mu1 element is then blunt-end ligated into the commercially available plasmid pGEM1 at the EcoRI site of the polylinker present in pGEM1 to produce the plasmid pM10. The plasmid pGEM1 contains the SP6 promoter and T7 promoter in opposite orientations so that either strand of the DNA between the promoters may be transcribed. This plasmid further contains a polylinker sequence.

The plasmid pM10 is then digested with PstI and SacII. The single-stranded ends are polished using Klenow fragment of DNA polymerase, and religated. This produces pM11, which contains a unique HindIII site and a unique AvaI site. The plasmid pM13 can be prepared by cleaving pM11 with AvaI and ligating with a polylinker sequence, i.e., multiple cloning site. pM10, pM11, pM13 or other recombinant vectors prepared in accordance with the present invention can be used to co-transform plants as described further below. They can also be used for the insertion of foreign DNA, to produce a plant transformation vector.

The foreign DNA can be inserted directly within the DNA sequence of the Mu1 element, preferably at a unique restriction site. Such sites include BstE11, BstNI and preferably AvaI. Each of these sites disrupt two of the four putative open reading frames of the Mu1 element. Alternatively, a multiple cloning site is first inserted within the DNA sequence of the Mu1 element, preferably at a unique restriction site. The multiple cloning site is a DNA fragment which contains a DNA sequence having the restriction sites of several restriction enzymes. In one embodiment, the multiple cloning site contains restriction sites for the following enzymes: PstI, Sal I, AccI, HincII, XbaI, BamHI and AvaI. The multiple cloning site preferably contains sites for restriction enzymes which are not present in the rest of the plant transformation vector. The foreign DNA is then inserted into one of the sites in the multiple cloning site.

In a second embodiment, the foreign DNA is inserted into the vector outside the DNA sequence for the Mu1 element. The foreign DNA is positioned on either the 5' or 3' side of the Mu1 element as it is found in the plant transformation vector. The plasmid pM11 contains a unique HindIII site outside the Mu1 element which can be used for the insertion of foreign DNA.

The foreign DNA is generally found in the vector in the form of a chimeric gene. The chimeric gene is constructed so that it comprises a promoter, which is derived from a gene expressible in the plant to be transformed and the desired sequence to be expressed. It is also preferred that the chimeric gene contains a 3' non-translated region containing a polyadenylation site of a gene which is expressible in the plant to be transformed. The chimeric gene may also contain additional regulatory sequences such as enhancers and the like, which are derived from a gene expressible in the plant to be transformed. An example of an enhancer-like element in plants has been described in Timko, M. P., et al., Nature 318, 579 (1985). Promoters which may be used include Ti plasmid-derived promotors such as Pnos and PTR, plant promoters such as Pssu pea, Pssu 301 and Pssu soybean, or plant virus promoters such as P35S from cauliflower mosaic virus. Tissue-specific promoters may be isolated by identifying genes preferentially expressed in particular tissue of the plant to be transformed and isolating the promoter region from the associated DNA sequence. The 3' non-translated region may also be derived from Ti plasmids such as the 3'-region of octopine synthase (OCS), plant genes or plant viral genes.

The desired sequence to be expressed is preferably placed in the chimeric gene construct so that there are no 5'-ATG codons between the promoter and start of the desired sequence. This will ensure that transcription will begin at the desired site. The chimeric gene is constructed using techniques known in the art, such as those described in the references cited above. The chimeric gene may be constructed within the vector or may be constructed and then inserted into the vector. The plant transformation vector which is used to transfer the foreign DNA to the desired plant will also preferably contain a marker if the foreign DNA is not a marker itself or readily assayable, such as in an immunoassay. The marker may be a selectable marker, i.e., one allowing for selection on a particular medium, or a scorable marker, i.e., one which produces a substance which can be measured. An example of a selectable marker is antibiotic resistance, such as kanamycin resistance. A suitable gene for kanamycin resistance is the neomycin phosphotransferase II (NPT II) gene. Examples of a scorable marker are the presence of octopine or acetylated chloramphenical derivatives. The appropriate genes are octopine synthase or chloramphenical acetyltransferase. The marker should be expressible in plant tissue so that transformation can be easily determined. Thus, the marker gene will contain a promoter which is derived from a gene expressible in the plant to be transformed.

In the preferred embodiment, the marker is the NPT II gene which confers resistance to kanamycin and G418. It has been discovered that kanamycin induces albinism in developing seedling plants when it is used on plant tissues such as embryos, embryogenic tissue or regenerable tissue. If the tissue is resistant to kanamycin, albinisim is prevented. The plant tissue subjected to transformation is transferred to medium containing kanamycin, and plants regenerated. Green plants indicate the expression of the kanamycin resistance gene. This allows for screening of transformed tissue at sublethal concentrations of kanamycin.

The marker is preferably placed on the 3' side of the foreign DNA if both are present. As with the foreign DNA, the marker preferably contains a plant expressible promoter and a 3' non-translated region containing a polyadenylation site of a plant expressible gene.

In one embodiment, a plant transformation vector pM14 is prepared in which a chimeric gene is inserted in the Aval site of pM11. The chimeric gene comprises the nopaline synthase promoter, the neomycin phosphotransferase structural gene, and the 3'-nopaline synthase fragment.

In a second embodiment, a plant transformation vector pM12 is prepared in which a polylinker sequence, the neomycin phosphotransferase structural gene, and the 3'-nopaline synthase fragment has been inserted at the Aval site of pM11. Promoters or 5'-DNA sequences isolated from plant tissues can be inserted in the polylinker sequence.

The foreign DNA is inserted into the vector in its entirety. That is, the chimeric gene is inserted into the plant transformation vector such that the entire chimeric gene is positioned within the DNA sequence of the Mu1 element or such that the entire chimeric gene is positioned outside the DNA sequence of the Mu1 element and on either its 5' or 3' side. Thus, the Mu1 element does not break up the chimeric gene. When the chimeric gene is positioned outside the Mu1 element, it is positioned adjacent the Mu1 element so that the transfer of the chimeric gene can be enhanced by the Mu1 element. Generally, "adjacent the Mu1 element" means that the chimeric gene will be positioned within 500 bases of either end of the Mu1 element. For example, a chimeric gene comprising the nopaline synthase promotor, the neomycin phosphotransferase structural gene, and the 3'-nopaline synthase fragment can be isolated and inserted into the HindIII site in pM11 or into any one of the restriction sites in the polylinker sequence in pM10.

The plasmids specifically described herein, namely pM10, pM11, pM12 and pM14, may be further modified to contain additional DNA sequences including foreign DNA, markers, polylinkers, and the like, to delete extraneous DNA sequences or any other alteration which is within the skill in the art. The Mu1 element and foreign DNA, if present, can be isolated from these plasmids and inserted into other cloning vectors, which is within the skill in the art.

Plants are transformed by any procedure known in the art for the introduction of plasmids into plant protoplasts, cells or tissues. Such procedures include micro-injection, in planta injection, electroporation, and treatment with CaCl₂ or similar agents. The treated plant cells or tissues are cultured to enable the transfer of the foreign DNA from the plant transformation vector to the genome. It is expected that the transposable element function of Mu1 will enhance the frequency of the foreign DNA transfer. Preliminary evidence indicates that the enhancement of transformation by the Mu1 element occurs when it is intact. Consequently, it is preferable that the foreign DNA be inserted adjacent the Mu1 element in the plant transformation vector as described above. If the foreign DNA is inserted within the Mu1 element, it is preferred to co-transform the plant protoplasts, cells or tissues with a mixture of a plant transformation vector having foreign DNA inserted within the Mu1 element and a recombinant vector containing the Mu1 element, such as pM10, pM11, or modifications thereof. The recombinant vector can also be used to co-transform plants with a vector which does not contain the Mu1 element but which does contain foreign DNA.

The plant cells or tissues are then subjected to a regeneration procedure to obtain plants. The regeneration may proceed via an embryogenic or organogenic route, and generally involves the formation of callus, followed by shoot and root formation. During the regeneration procedure, the plant tissue is screened for transformed plants. It is preferred to screen for transformed plants by selecting between green plants and albino plants as previously described. Transformed plants are then examined to determine if they contain the foreign DNA via blot hybridization, DNA analysis, gene expression or other suitable technique.

The present invention will be further described by reference to the following non-limiting examples. DNA manipulations were carried out in accordance with manufacturers' recommended procedures, Maniatis, T., et al., supra, or Miller, J. H., supra, unless indicated otherwise. All enzymes were obtained from BRL, New England Biolabs or International Biotechnology, Inc.

## EXAMPLE 1

### Preparation of Stock Solutions

#### A. MS Salts

A 100X stock solution of MS salts was prepared by dissolving 37.0 g magnesium sulfate heptahydrate, 44 g calcium chloride dihydrate, 17 g monopotassium phosphate, 190 g potassium nitrate, 165 g ammonium nitrate, 620 mg boric acid, 1.69 g manganese sulfate monohydrate, 860 mg zinc sulfate heptahydrate, 25 mg sodium molybdate (VI) dihydrate, 2.5 mg copper (II) sulfate pentahydrate, 2.5 mg cobalt chloride hexahydrate, 83 mg potassium iodide, 2.78 g iron (II) sulfate heptahydrate and 3.73 g disodium-EDTA in one liter of distilled, deionized water.

#### B. Vitamins

A 100X stock solution of vitamins was prepared by dissolving 0.1 mg biotin, 10 mg pyridoxine hydrochloride, 10 mg nicotinic acid, 10 mg thiamine hydrochloride, 10 mg calcium pentothenate, and 10 mg L-cysteine chlorohydrate in 100 ml of distilled, deionized water.

#### C. Buffers

10X stock solutions of the following buffers were prepared by dissolving the specified components in 100 ml of distilled, deionized water to give the specified concentrations of the components.

| Component | Concentration |
|---|---|
| **i.** Tris Borate Electrophoresis Buffer | |
| Tris Base | 0.90 M |
| Boric Acid | 0.88 M |
| EDTA | 25 mM |
| **ii.** Restriction Buffer | |
| Tris pH 7.4 | 250 mM |
| $MgCl_2$ | 100 mM |
| 2-mercaptoethanol | 100 mM |
| Bovine Serum Albumin | 100 µg/ml |
| **iii.** Nick Translation Buffer | |
| Tris pH 7.4 | 0.5 M |
| $MgCl_2$ | 100 mM |

## EXAMPLE 2

### Preparation of Solutions

A. Solution I

Solution I was prepared by dissolving 91.1 g of mannitol and 10 mg of benzylaminopurine (BAP) in 1 liter of distilled, deionized water. The solution was sterilized by passing through a 0.22 μ Millipore membrane and the pH adjusted to 5.5, using either 1N HCl or 1N NaOH.

B. Solution II

Solution II was prepared by dissolving 91.1 g of mannitol, 10 mg of BAP, 15 g of cellulase "Onozuka" RS (obtained from Yokult Honsha Co. Ltd.) and 6 gm of macerozyme R-10 (obtained from Yokult Honsha Co. Ltd.) in 1 liter of distilled, deionized water. The solution was sterilized by passing through a 0.22 μ Millipore membrane and the pH adjusted to 5.5, using either 1N HCl or 1N NaOH.

C. Solution III

Solution III was prepared by dissolving 171.2 g of sucrose in 1 liter of distilled, deionized water. The solution was sterilized and the pH adjusted to 5.5, as described above.

D. Solution IV

Solution IV was prepared by dissolving 80.16 g of mannitol 20.54 g of sucrose, 100 mg of inositol, 0.2 mg of BAP, 0.1 mg of 2,4-dichlorophenoxyacetic acid (2,4-D), 1 mg of α-naphthalene acetic acid (NAA), 10 ml vitamin stock solution, and 10 ml MS salt stock solution and 980 ml of distilled, deionized water. The solution was sterilized and pH adjusted as previously described.

E. Solution V

Solution V was prepared by dissolving 80.16 g of mannitol, 20.54 g of sucrose, 100 mg of inositol, 0.3 mg of BAP, 200 mg of casein hydrolysate (Difco), 20 mg of sodium pyruvate, 10 ml vitamin stock solution, and 10 ml MS salt stock solution in 980 ml of distilled, deionized water. The solution was sterilized and pH adjusted as previously described. Solution V with selection pressure was also prepared by adding 200 mg/l of kanamycin.

F. Electroporation Buffer

Electroporation buffer was prepared by dissolving 8 g of NaCl, 0.2 g of KCl, 0.2 g of $Kh_2PO_4$ and 1.15 g of $Na_2HPO_4$ in 1 liter of distilled, deionized water. After diluting the mixture 1:1 with distilled, deionized water, mannitol was added to a final concentration of 0.2 M. The pH was adjusted to 5.5 with 1N HCl.

G. Stet Buffer

Stet buffer was prepared by dissolving 8 g of sucrose in 78.5 ml of distilled, deionized water and adding 500 μl of Triton X-100, 20 ml of a 0.25 M solution of EDTA, pH 8.0, and 1 ml of a 1M solution of Tris pH 8.0.

## EXAMPLE 3

### Preparation of Solid Media

#### A. Callus Proliferation Medium

Callus proliferation medium was prepared by adding one package of Murashige and Skoog salt mixture (Gibco Laboratories Catalog No. 510-3118), 20 g of sucrose, 0.2 mg of benzyladenine (BA), 1 mg of NAA and 2 g of gelrite to 1 liter of distilled, deionized water. The medium was sterilized by autoclaving for 15 minutes at 240°F and 15 psi. A callus proliferation medium with selection pressure was prepared by adding 200 mg of kanamycin to the mixture after sterilization.

#### B. Regeneration Medium

Regeneration medium was prepared as described above, except that 2 mg of BAP was added in place of the BA and NAA. Regeneration medium with selection pressure was prepared as described above.

#### C. Plantlet Establishment Medium

Plantlet establishment medium was prepared as in A above, except that 1 mg of BAP and 0.1 mg of indole-3-butyric acid (IBA) was added in place of BA and NAA. Plantlet establishment medium with selection pressure was prepared as described above, except that 75 mg of kanamycin was used.

#### D. Selection Medium

Selection medium was prepared by adding one package of Murashige and Skoog salt mixture, 10 g of sucrose, and 2 g of Gelrite to 1 liter of distilled, deionized water. The medium was sterilized as described above, and 200 mg of kanamycin was then added.

## EXAMPLE 5

The following general procedures were utilized in the examples which follow.

#### A. Boiling Mini-Prep

Single colonies were grown to stationary phase in 5 ml of the appropriate medium. 1.5 ml was then transferred to microfuge tubes and centrifuged for 5 minutes. The supernatant was removed with an aspirator and the pellet resuspended in 350 μl of Stet Buffer. 25 μl of fresh lysozyme (10 mg/ml in 10 mM Tris pH 8) was added, and the mixture was vortex mixed and held at room temperature for 5 minutes. The tubes were then placed in a boiling waterbath for 40-60 seconds and immediately quenched in an ice waterbath for 1 minute. The mixture was centrifuged at room temperature for 15 minutes, after which the pellet was removed with a sterile toothpick and discarded. 40 μl of 2.5M sodium acetate and 420 μl of isopropanol were added to the supernatant and mixed well. The mixture was placed in a dry ice/ethanol bath for 15 minutes. A pellet was obtained by centrifugation at 4°C for 15 minutes. The pellet was dried by lyophilization and resuspended in 20 μl of 10mM Tris pH 7.4. RNA background was removed, if desired, by adding 50 μg/ml of RNase to the buffer.

## B. Transformation

Host bacteria such as HB101 was grown in 10 ml of overnight culture in appropriate media, such as L-broth. 100 ml of media containing 20 mM $MgCl_2$ was inoculated with 1 ml of overnight culture, and the bacteria grown with vigorous shaking at 37°C to a cell density of $5 \times 10^7$ cells/ml, usually 2-3 hours. The cells were then transferred to a sterile 500 ml centrifuge bottle and chilled on ice. The 10 ml of cells were centrifuged in snap cap tubes for 10 minute at 5,000 rpm (4000 xg) at 4°C. The cells were resuspended in 2.5 ml of a transformation buffer and held on ice for 15 minutes. The cells were recentrifuged and the pellet resuspended in 600 μl of transformation buffer. 200 μl aliquots were dispensed into sterile, pre-chilled glass tubes and stored at 4°C for 12-24 hours.

Three separate transformations were conducted for recombination selection. Supercoiled and self-annealed samples of plasmid DNA were used as controls for transformation fidelity. 40 ng of DNA in either Ligation Buffer or TE buffer were added to a single aliquot of cells, mixed very gently, and held on ice for 30 minutes. The tubes were transferred to a 41°C water bath and held for 2 minutes. 800 μl of LB were then added, and the tubes incubated at 37°C for 30 minutes or for 60 minutes when selecting with ampicillin or kanamycin. The non-transformed competent cells were titrated during the incubation to determine viable cell titer. This was performed by spreading 100 μl aliquots of 10-4 to 10-8 dilutions onto LB plate (100 μl LB + 11 μl cells = 10-1). Some of the lower dilutions were placed onto selective plates as a control. After incubation, 500 μl aliquots of the transformed cells were spread onto selective plates (20 for the entire mixture). Supercoiled transformants were plated at a 1-10 dilution. The plates were incubated overnight at 37°C and the transformation efficiency and frequency were then calculated.

## C. Electroelution

The DNA band was physically sliced from a 1% agarose gel (usually preparative). The gel slice was placed in a dialysis tubing bag containing a minimum volume of 0.5X Tris-Borate Electrophoresis Buffer. The bag was submerged in the same buffer in an electrophoresis apparatus and electrophoresed for 3-4 hours at 100 volts. The electrode terminals were then reversed and 100 volts applied for 1 minute to release any DNA stuck to the bag. The bag was then removed from the electrophoresis apparatus.

## D. Ethanol Precipitation

After electroelution, the buffer was removed from the bag and ammonium acetate was added to a final concentration of 300 mM. 2 volumes of cold absolute ethanol were added and the DNA was precipitated at -20°C overnight or at -70°C in a dry ice/ethanol bath for 15 minutes. The DNA was pelleted at 12,000 xg. The pellet was dried under vacuum and the DNA was resuspended in either distilled, deionized water or 10mM tris pH 7.4 at a concentration of 500 μg/ml.

## EXAMPLE 5

### Construction of pM10 and pM11

A mixture of 350 μl containing 70 μg of pMJ9 (obtained from M. J. Freeling), 45 μl of 0.5M NaCl, 45 μl of 10X Restriction Buffer, and 10 μ of PvuII (100 units) was incubated for 1 hour at 37°C. Then 15 μl of 10X Restriction Buffer, 55 μl of 0.5M NaCl, 55 μl of water, 5 μl of HindIII (100 units), and 20 μl of Sau3a (40 units) were added. The mixture was incubated for 1 hour at 37°C. A 1% agarose preparative gel was then run and the Mu1 element fragment was isolated by electroelution as described above.

A mixture of 20 μl containing 10 μg of pGEM1, 2 μl of 0.5M NaCl, 9μl of water, and 5 μl of EcoRI (20 units) was incubated at 37°C for 1 hour. The DNA was extracted first with phenol:chloroform (24:1) and then with chloroform. The DNA was ethanol precipitated, and resuspended in 20 μl of water.

The Eco RI restricted pGEM1 and Mu1 element fragment were treated to polish ends for blunt end ligation. This was accomplished by incubating for 1 hour at 30°C a mixture containing 1-10 μg of DNA, 1X Nick Translation Buffer, 1mM each of dCTP, dGTP, dTTP and dATP, and 1-2 units/μg DNA of Klenow fragment of DNA polymerase I. The final volume of the reaction mixture was dictated by the original concentration of the DNA and adjusted to the final desired concentration by the addition of 10X Nick Translation Buffer, 10 mM solution of nucleotides, and water. Following incubation of the Mu1 element fragment only, 50 units/µg DNA of bacterial alkaline phosphatase were added and incubated an additional 1 hour at 65°C. Both DNA preps were phenol extracted, ethanol precipitated, and the DNA was resuspended in water.

The EcoRI restricted pGEM1 and Mu1 element fragment were blunt-end ligated by incubating a mixture of 6.7 µl containing 1 µg of EcoRI restricted pGEM1. 5µl containing 500 ng of the Mu1 element fragment, 2 µl of 10X Ligation Buffer, 4 µl of 10 mM rATP and 2.3 µl T4 ligase (approximately 5 units) at 12°C overnight. Competent E. coli HB101 was transformed as described above. The transformants were identified by restriction analysis after a Boiling Mini-Prep as described above. A plasmid was identified which contained the 1400 bp Mu1 element inserted into the EcoRI site of pGEM1 and named pM10.

pM11 was produced by removing the polylinker from pM10. This was accomplished by restricting pM10 with SacI and PstI. A mixture of 10 µl containing 5 µg of pM10, 1.5 µl of 10X Restriction Buffer, and 3.5 µl of SacI (15 units) was incubated for 1 hour at 37°C. Then, 1µl of 10X Restriction Buffer, 2 µl of 0.5M NaCl and 2µl of PstI (10 units) was added. The mixture was incubated at 37°C for 1 hour. The DNA was extracted with phenol, ethanol precipitated, and resuspended in water. The restricted pM10 was treated to polish the ends as described above. The DNA was then ethanol precipitated and resuspended in 10 µl of 10 mM Tris pH 7.4. The ends were religated by incubating overnight at 12°C a mixture of 5µl containing 2.5 µg of restricted and polished pM10, 1 µl of 10X Ligation Buffer, 2 µl of 10mM rATP, and 2 µl of T4 ligate. E. coli HB101 was transformed and colonies screened for the absence of BamHI sites.

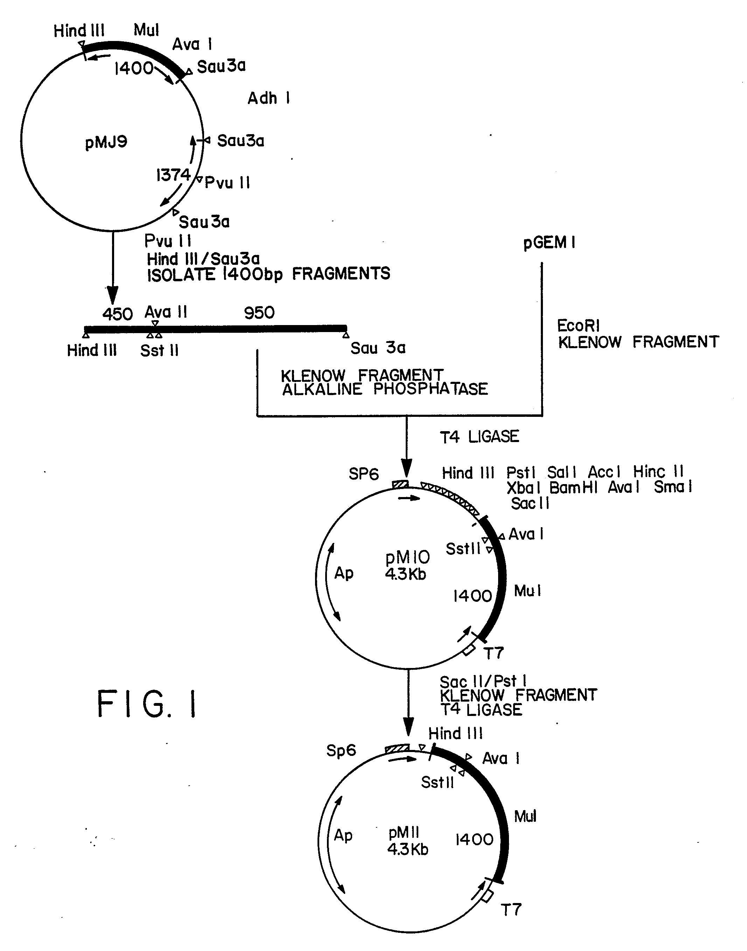

Figure 1 illustrates the construction of pM10 and pM11. The closed box represents the Mu1 element, the open box represents the T7 promoter, and the hatched box represents the SP6 promoter. The ampicillin gene is shown by Ap. Restriction sites are indicated for each vector.

E. coli HB101 containing pM10 and E. coli HB101 containing pM11 were deposited at the American Type Culture Collection (ATCC), Rockville, Maryland, U.S.A. under the Budapest Treaty on April 16, 1986, and assigned numbers 67086 and 67087, respectively.

pM11 can be modified by inserting a polylinker sequence, i.e., multiple cloning site, at one of the unique restriction sites within the Mu1 element, preferably at the AvaI site.


## EXAMPLE 6

### Construction of pM12

A mixture of 20 µl containing 5 µg of pM11, 3 µl of 10X Restriction Buffer, 3 µl of 0.5M NaCl, and 4 µl of AvaI (24 units) was incubated for 1 hour at 37°C. The DNA was extracted with phenol, ethanol precipitated, and resuspended in water. The restricted pM11 was treated to polish the ends as described above, and then treated with bacterial alkaline phosphatase as previously described.

The plasmid pNN was restricted with a mixture of HindIII and SacI in a standard manner, as recommended by the enzyme suppliers and as performed herein. A 1% agarose preparative gel was run and the HindIII/ SacI fragment containing the neomycin phosphotransferase structural gene and the 3'-nopaline synthase fragment was isolated by electroelution. The isolated fragment was treated to polish the ends as previously described.

The restricted pM11 and HindIII/SacI fragment were blunt-end ligated by incubating a mixture of 5 µl containing 500 ng restricted pM11, 5 µl containing 1 µg of the HindIII/SacI fragment, 2 µl of water, and 2 µl of T4 ligase overnight at 12°C. E. coli HB101 was transformed and colonies screened for the correct orientation of the HindIII/SacI fragment. One plasmid with the proper orientation was identified as pM12.

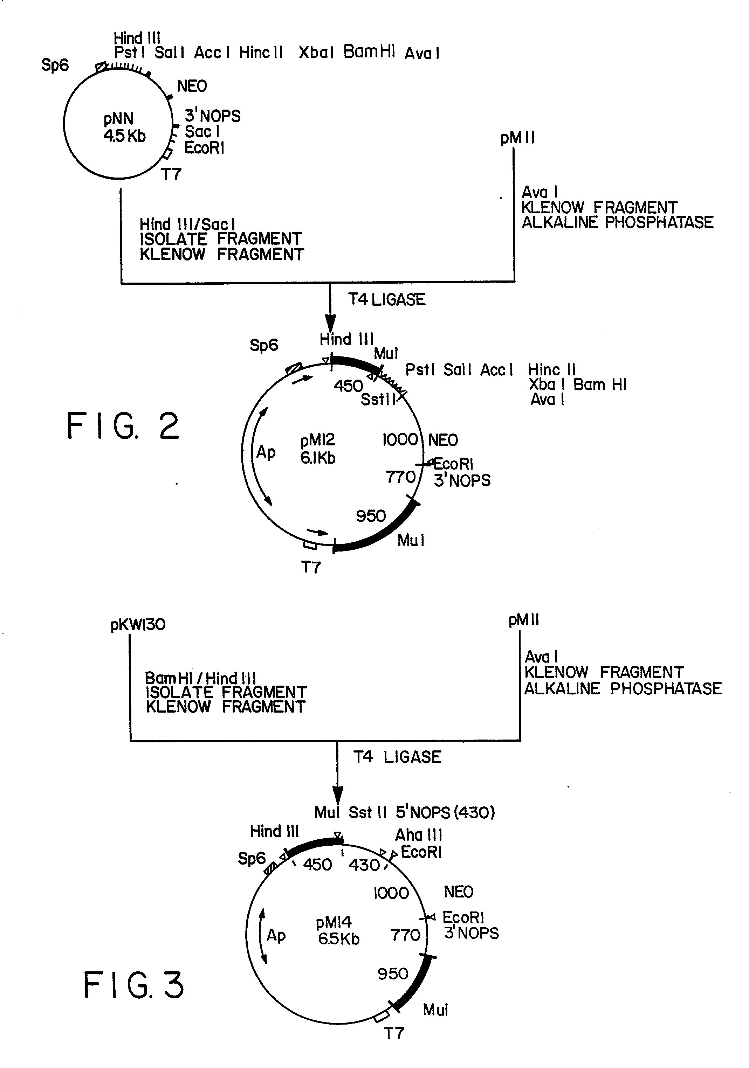

Figure 2 illustrates the construction of pM12. The closed box represents the Mu1 element, the open box represents the T7 promoter, and the hatched box represents the SP6 promoter. The ampicillin resistance gene, neomycin phosphotransferase structural gene, and 3'-nopaline synthase fragment are shown by Ap, Neo and 3'-NOPS, respectively.

E. coli HB101 containing pM12 was deposited at the ATCC under the Budapest Treaty on April 16, 1986 and assigned number 67088.

## EXAMPLE 7

### Construction of pM14

pM11 was restricted with Aval, and the ends polished and treated with phosphatase as described in Example 6.

A mixture of 70 μl containing 50 μg of pKW 130, 15 μl of 10X Restriction Buffer, 30 μl of 0.5M NaCl, 15 μl of water, 10 μl of BamHI (200 units), and 10 μl of HindIII (200 units) was incubated at 37°C for 1 hour. A 1% agarose preparative gel was run, and the BamHI/HindIII fragment containing the 5'-nopaline synthase fragment, the neomycin phosphotransferase structural gene and the 3'-nopaline synthase fragment was isolated by electroelution. The isolated fragment was treated to polish the ends as previously described. During the restriction reaction a deletion occurred in the 5'-nopaline synthase fragment found in pKW 130, so that only a 430 bp fragment was recovered.

The restricted pM11 and BamHI/HindIII fragment were blunt-end ligated as described in Example 6 except that the BamHI/HindIII fragment was used in place of the HindIII/SacI fragment. E. coli HB101 was transformed and colonies screened for the correct orientation of the BamHI/HindIII fragment. One plasmid with the proper orientation was identified as pM14.

Figure 3 illustrates the construction of pM14. The closed box represents the Mu1 element, the open box represents the T7 promoter, and the hatched box represents the SP6 promoter. The ampicillin resistance gene, 5'-nopaline synthase fragment, neomycin phosphotransferase structural gene, and 3'-nopaline synthase fragment are shown by Ap, 5'-NOPS, Neo and 3'-NOPS, respectively.

E. coli HB101 containing pM14 was deposited at the ATCC under the Budapest Treaty on April 16, 1986 and assigned number 67089.

## EXAMPLE 8

### Transformation of Tobacco

#### A. Protoplast Preparation

3-4 cm long tobacco leaves (Nicotiana tabacum c.v. Havana) were taken from in vitro growing plants for protoplast isolation. The upper epidermis of the leaves was brushed with 320 grit aluminum oxide particles to allow enzyme infiltration. The leaves were rinsed with sterile water to wash off the abrasive powder. Then the leaves were placed brushed surface down on plasmolysis solution (Solution I) for 60 minutes. Next, Solution I was replaced by an enzyme mixture (Solution II) for overnight digestion. The leaves generally float during the progress of digestion. After digestion, Solution II was carefully pipetted out and Solution III was dispensed on top of the leaves to disperse, wash and release the protoplasts. Mesophyll protoplasts were floated and harvested after centrifugation for 5 minutes at 300 xg in a 50 ml babcock bottle.

#### B. Electroporation Procedure

A 2.8 ml multi-drop plate was prepared with pre-chilled sterile electroporation buffer mixed with 100 μg of pM14 and 6 μg pM11. Protoplasts were dispensed and mixed with the solution to a density of $1 \times 10^6$ cells/ml. One pulse of current from an electrode with a distance of 1 cm between poles was discharged out of a capacitor (200 volt, 780μF) into the cell mixture for approximately 50 milliseconds. The cell mixture was pipetted out and washed with 10 ml of Solution III in a 15 ml conicle tube. A 1 ml volume of solution IV was overlaid on top of the mixture. The mixture was then centrifuged at 200 xg for five minutes to separate viable undamaged cells which remain in the supernatant. Removal of the damaged or dead cells increases cell survival and viability.

The undamaged cells were then plated with Solution IV at a density of $1 \times 10^5$ cells/ml. After three days, density was lowered to $1 \times 10^4$ cells/ml by adding Solution IV. After 0.1 mm cell aggregates formed, a 1:1 dilution was made by adding Solution V. After the cell aggregates had reached a size of 0.5 mm, the aggregates were spun down by centrifugation at 200 xg for two minutes. The aggregates were mixed with 2.5 ml of Solution V. The aggregate solution was then plated onto selection medium and maintained for two to three weeks. Then Solution V, containing 200 mg/l of kanamycin, was added to replenish selection pressure and cultured until resistant colonies formed.

Alternatively, after the cell aggregates reached the size of 0.5 mm, kanamycin was added to a concentration of 200 mg/l. After two weeks, this solution was concentrated to 2.5 ml by centrifugation as described above, and then plated onto selection medium and cultured until resistant colonies formed.

## C. Regeneration and Analysis

Resistant colonies were plated on callus proliferation medium with selection pressure and cultured for 2-3 weeks at 24°C in a 16 hour diffused light/8 hour dark cycle. Callus was subcultured every 2-3 weeks to produce enough tissue to proceed with regeneration. After enough tissue was obtained, the callus was transferred to regeneration medium with or without selection pressure and cultured for 3-4 weeks at 24°C in a 16 hour diffused light/8 hour dark cycle until shoot bud formation. At this time, the material was transferred to plant establishment medium with or without selection pressure and cultured at 24°C in a 16 hour diffused light/8 hour dark cycle until 3-4 leaves formed. The plantlet was then transferred to soil. A leaf section from the transformed plant was also plated on callus proliferation medium with selection pressure.

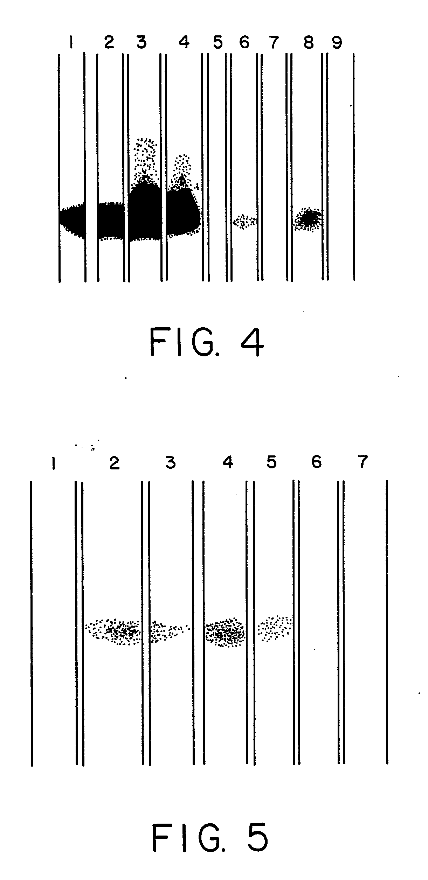

The transformed tissue as well as controls were assayed for kanamycin resistance according to the procedure of Fregien et al., Anal.Biochem. 148, 101 (1985). The results are shown in Figures 4 and 5. In Figure 4, the following bands are illustrated. 1-4 are E. coli containing pNEO (to assay for activity), 5 is an E. coli control, 6 is callus from tobacco transformed with pM14, 7 is callus from tobacco transformed with pABD1 (which contains the neomycin phosphotransferase gene), 8 is tissue from crown gall infection of sunflower by Agrobacterium containing a Ti plasmid having a neomycin phosphotransferase gene, and 9 is control tobacco callus.

The following bands are illustrated in Figure 5. 1 is control tobacco callus, 2 is pM14 transformed tobacco callus selected on kanamycin, 3 is pM14 transformed tobacco callus removed from kanamycin selection, 4 is leaf from transformed plant grown on kanamycin, 5 is leaf from plant removed from kanamycin, 6 is control tobacco leaf and 7 is control tobacco plant exposed to kanamycin at 75 µg/ml.

These results demonstrate that the pM14 is capable of transforming tobacco protoplasts, and that the neomycin phosphotransferase gene is integrated into the tobacco genome and expressed constitutively in tobacco tissue.

## EXAMPLE 9

## Transformation of Corn Protoplasts

### A. Protoplast Preparation

A B73 corn plant approximately 3 feet tall was cut at the part of the stem with the expanding foliage. The top three layers were peeled off and the stem wiped with 75% ethanol. The next three layers were peeled off under sterile conditions and the stem was then cut in half. Strips of leaves were removed from the inside to the outside. The leaves were then treated, and protoplasts harvested as described for tobacco protoplasts in Example 8.

### B. Electroporation

The corn protoplasts were transformed by electroporation as described in Example 8, except that only pM14 was utilized instead of a mixture of pM14 and pM11. Undamaged cells were obtained as described in Example 8. The undamaged cells were cultured in Solution IV for 48 hours. The cells were isolated by centrifugation for 5 minutes at 200 xg and assayed for transient expression of the neomycin phosphotransferase gene.

While the invention has been described in connection with specific embodiments thereof, it will be understood that it is capable of further modifications. This application is intended to cover any variations, uses or adaptations of the invention following, in general, the principles of the invention and including such departures from the present disclosure as come within known and customary practice within the art to which the invention pertains.

13

## Claims

1. A plant transformation vector comprising the Mu1 element and foreign DNA, wherein the foreign DNA is positioned entirely either (a) within the Mu1 element, or (b) adjacent the Mu1 element.

2. The plant transformation vector of claim 1 wherein the foreign DNA is a chimeric gene comprising a plant expressible promoter and a DNA sequence of a structural gene.

3. The plant transformation vector of claim 2 wherein the chimeric gene further comprises a plant recognizable 3'-nontranslated DNA sequence.

4. The plant transformation vector of any one of the preceding claims which further comprises a marker, wherein the marker is positioned entirely within the Mu1 element.

5. The plant transformation vector of any one of claims 1-3 which further comprises a marker, wherein the marker is positioned adjacent the Mu1 element.

6. The plant transformation vector of claim 4 or 5 wherein the marker comprises a plant expressible promoter, a DNA sequence of the structural gene for the marker, and a plant recognizable 3'-nontranslated DNA sequence.

7. A vector seelcted from the group consisting of pM10 (ATCC 67086), pM11 (ATCC 67087), pM12 (ATCC 67088), pM14 (ATCC 67089), or modifications thereof.

8. A recombinant DNA vector useful in the transformation of plants which comprises a cloning vector having covalently bound thereto a DNA insert consisting essentially of the DNA sequence of the Mu1 element of maize.

9. A method for transforming plants which comprises introducing the plant transformation vector of any one of the preceding claims into plant cells, protoplasts or tissues, culturing said protoplasts, cells or tissues and regenerating transformed plants from said protoplasts, cells or tissues.

10. A method for transforming plants which comprises introducing the plant transformation vector of any one of claims 1-7 and a recombinant DNA vector, said recombinant DNA vector comprising a cloning vector having covalently bound thereto a DNA insert consisting essentially of the DNA sequence of the Mu1 element of maize, into plant cells, protoplasts or tissues, culturing said protoplasts, cells or tissues and regenerating transformed plants from said protoplasts, cells or tissues.

11. The method of claim 10 wherein said recombinant DNA vector is selected from the group consisting of pM10 (ATCC 67086), pM11 (ATCC 67087), or modifications thereof.

12. A method for transforming plants which comprises introducing the recombinant DNA vector of claim 8 and a plant transformation vector, said plant transformation vector comprising a cloning vector having covalently bound thereto a DNA insert comprising foreign DNA, into plant cells, protoplasts or tissues, and regenerating transformed plants from said protoplasts, cells or tissues.

FIG. I

FIG. 2

FIG. 3

FIG. 4

FIG. 5

Application number

## DOCUMENTS CONSIDERED TO BE RELEVANT

EP 86307774.9

| Category | Citation of document with indication, where appropriate, of relevant passages | Relevant to claim | CLASSIFICATION OF THE APPLICATION (Int. Cl.4) |
|---|---|---|---|
| D,X | NUCLEIC ACIDS RESEARCH, vol. 12, no. 15, August 10, 1984, London<br>R.F.BARKER et al. "Nucleotide sequence of the maize transposable element MuI"<br>pages 5955-5967<br>  * Abstract; page 5959 *<br>    ----| 1,8 | C 12 N 15/00<br>A 01 H 1/00 |

**TECHNICAL FIELDS SEARCHED (Int. Cl.4)**

C 12 N

A 01 H

The present search report has been drawn up for all claims

| Place of search | Date of completion of the search | Examiner |
|---|---|---|
| VIENNA | 31-07-1987 | WOLF |